# FASCICULE DE BREVET EUROPEEN

(11) **EP 0 687 208 B1**
(45) Date de publication et mention de la délivrance du brevet: **02.04.1997**
(21) Numéro de dépôt: 94908387.7
(22) Date de dépôt: 28.02.1994
(51) Int. Cl.: B23B 51/02, B23B 51/06, A61C 8/00, A61B 17/16

(54) **FORET POUR LA POSE D'UN IMPLANT DENTAIRE NOTAMMENT**
BOHRER, INSBESONDERE ZUM EINSETZEN EINES DENTALEN IMPLANTATS
DRILL, PARTICULARLY FOR INSERTING A DENTAL IMPLANT

(30) Priorité: 01.03.1993 FR 9302620
(43) Date de publication de la demande: 20.12.1995
(73) Titulaire: BORDES, Sylvain, F-33740 Mestras (FR)
(72) Inventeur: BORDES, Sylvain, F-33740 Mestras (FR)
(74) Mandataire: Martin, Jean-Jacques
(86) Numéro de dépôt international: FR9400224
(87) Numéro de publication internationale: WO9420247

(56) Documents cités:
- DE-A- 1 602 794
- GB-A- 2 086 279
- US-A- 3 460 410
- US-A- 4 135 847
- US-A- 5 100 267

## Description

Généralement, un implant dentaire comprend un corps impacté dans l'os alvéolaire. Pour cette impaction, il est nécessaire de procéder à un perçage de l'os au moyen d'un foret. Les forets employés pour ce genre d'opération, sont réalisés en métal, notamment en acier inoxydable et constituent des ensembles monoblocs.

Le coût de ces forets est élevé, compte-tenu de la nature des matériaux les consitutant et de la nécessité d'un usinage particulier pour constituer les arêtes de coupe. Ce coût se trouve être un élément important, étant donné qu'à chaque implant, correspond une instrumentation adaptée.

Par ailleurs, pour réutiliser de tels outils, il est nécessaire de les stériliser compte-tenu de leur application et éviter ainsi tout risque de contamination. Il est donc obligatoire, à chaque utilisation, de procéder à un nettoyage et à une stérilisation particulièrement soignés.

En outre, ces utilisations répétées engendrent inévitablement une usure de l'outil et par conséquent, une diminution de l'efficacité.

L'invention s'est fixée pour but de remédier à ces incovénients, de manière simple, sûre, efficace et rationnelle.

Le problème que se propose de résoudre l'invention, est de proposer un foret d'un coût réduit, et jetable après chaque utilisation, évitant ainsi les opérations de nettoyage et de stérilisation, en étant par conséquent, certain de l'aspect stérile à chaque utilisation.

Pour résoudre un tel problème et dans une première forme de réalisation, il a été conçu et mis au point un foret comprenant les caractéristiques de la revendication 1.

Pour résoudre le problème posé d'augmenter l'efficacité de la coupe et la dureté de la partie active, cette dernière présente un insert métallique faisant office d'arête tranchante.

On prévoit également que l'extrémité de la partie active présente une pastille en métal rapportée et noyée dans la matière plastique.

Pour résoudre le problème posé d'éviter tout échauffement osseux, la partie de raccordement est percée pour le passage d'un fluide lubrifiant, le trou débouchant dans au moins une gorge formée dans la partie active pour diriger le fluide jusqu'à la pointe du foret.

La partie active de coupe présente longitudinalement des rainures hélicoïdales dans lesquelles débouchent la ou les gorges.

Pour augmenter la rigidité de l'ensemble du foret et éviter tout effet de sicaillement, la partie active présente au niveau de son raccordement avec la tige, une portée cylindrique de plus grand diamètre.

L'invention est exposée, ci-après plus en détail à l'aide des dessins annexés, dans lesquels :

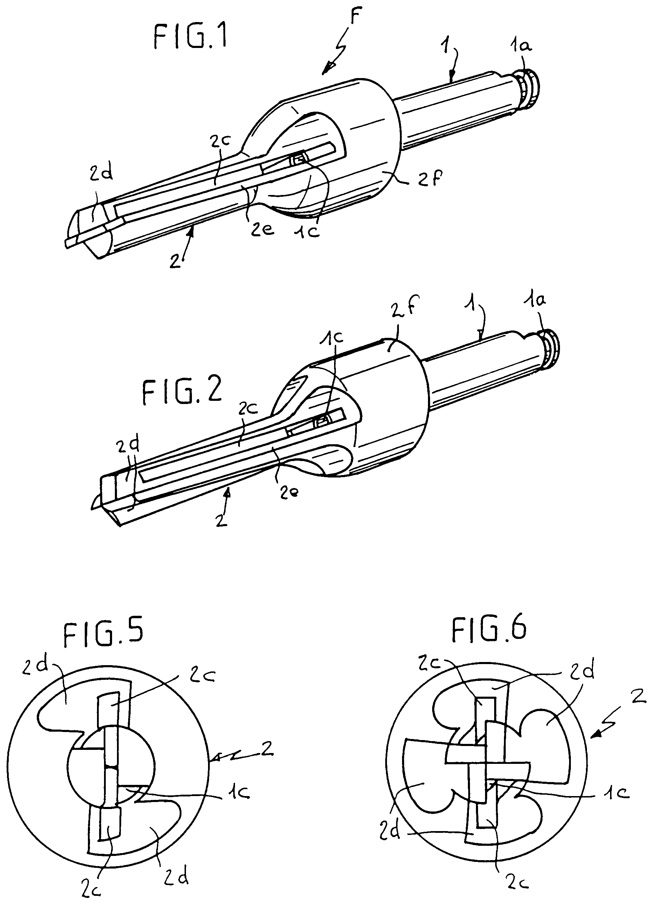

La figure 1 est une vue en perspective d'une forme de réalisation du foret selon l'invention.
La figure 2 est une vue en perspective d'une autre forme de réalisation du foret selon l'invention.
La figure 3 est une vue en coupe longitudinale du foret dans le cas où ce dernier est réalisé entièrement en matière plastique.
La figure 4 est une vue en coupe longitudinale d'une autre forme de réalisation du foret.
La figure 5 est, à une échelle plus importante, une vue en coupe transversale considérée selon la ligne 5.5 de la figure 1.
La figure 6 est, à une échelle plus importante, une vue en coupe transversale considérée selon la ligne 6.6 de la figure 2.

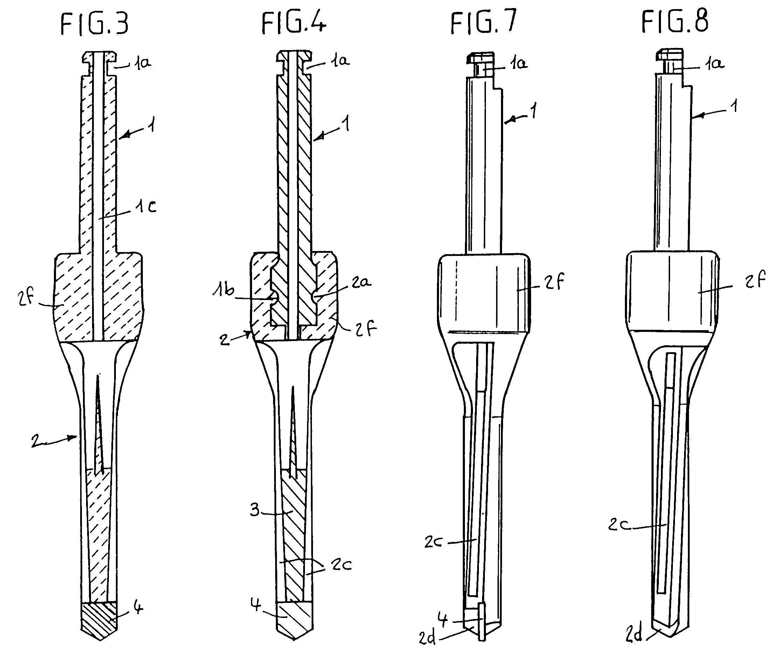

La figure 7 est une vue de face du foret correspondant à la forme de réalisation illustrée figure 1.
La figure 8 est une vue de face du foret correspondant à la forme de réalisation illustrée figure 2.

Le foret désigné dans son ensemble par (F) comprend une partie de raccordement (1) sous forme d'une tige et une partie active de coupe (2). La partie (1) est agencée à son extrémité libre (1a) pour être accouplée à tout type d'appareil du type perçeuse.

Dans la forme de réalisation illustrée figure 3, les parties (1) et (2) constituent un ensemble monobloc obtenu notamment par injection de matière plastique, afin de constituer un foret jetable.

Dans la forme de réalisation illustrée figure 4, les deux parties (1) et (2) sont indépendantes. La partie (1) constitue une tige en acier inoxydable. La partie (2) est en matière plastique. Les parties (1) et (2) présentent des agencements complémentaires (1b) et (2a) pour être assemblées par surmoulage. Par exemple, ces agencements sont constitués par des gorges périphériques formées en bout de la tige (1) constituant des zones de retenue de la partie active (2). Avec cette solution, le foret constitue également un ensemble jetable.

Dans cette forme de réalisation, la partie active (2) peut présenter un insert métallique (3) faisant office d'arête tranchante.

Quel que soit le mode d'exécution du foret, c'est-à-dire en constituant un ensemble monobloc entièrement en matière plastique (figure 3) ou bien un ensemble incluant à la fois des parties en matière plastique et des parties en métal, l'extrémité de la partie active (2) peut présenter une pastille de coupe (4). Cette pastille est surmoulée avec la matière plastique de la partie active (2) et est réalisée en métal, notamment en titane.

Suivant une autre caractéristique, la tige (1) est percée longitudinalement en (1c) pour le passage d'un fluide lubrifiant. Ce trou (1c) débouche dans au moins une gorge (2c) formée dans la partie active (2) pour diriger le fluide jusqu'à la pointe du foret. Compte-tenu de la conception en matière plastique de la partie active (2), cette dernière présente des rainures profilées (2d) délimitant des arêtes tranchantes (2e).

La partie active (2) présente au niveau de son raccordement avec la tige (1), une zone (2f) sous forme d'une portée cylindrique de plus grand diamètre et se raccordant d'une manière dégressive avec ladite partie (2). Cette portée (2f) constitue une zone de renforcement.

Les avantages ressortent bien de la description, en particulier on souligne et on rappelle :
- le caractère jetable du foret à chaque utilisation évitant tout nettoyage et stérilisation,
- l'irrigation interne du foret évitant tout échauffement osseux,
- la qualité de coupe obtenue du fait de l'utilisation d'un nouveau foret à chaque intervention,
- le coût de revient réduit.

## Revendications

1. Foret pour la pose d'implant dentaire notamment, comprenant une partie d'entraînement en rotation se présentant sous la forme d'une tige métallique (1), destinée à être raccordée par une première extrémité (1a) à un appareil du type perceuse, et une partie active de coupe (2) munie d'au moins une arête tranchante, caractérisé en ce que la partie active de coupe (2) est réalisée en matière plastique surmoulée sur la seconde extrémité de la partie d'entraînement en rotation, et en ce que ladite partie active de coupe (2) comporte un insert métallique (4) faisant office d'arête tranchante.

2. Foret selon la revendication 1, caractérisé en ce que la partie d'entraînement en rotation (1) et la partie active de coupe (2) comprennent des agencements de formes complémentaires (1b, 2a) aptes à assurer la solidarisation des deux parties en rotation.

3. Foret selon l'une des revendications 1 ou 2, caractérisé en ce que l'insert métallique de la partie active de coupe (2) est une pastille (4) en métal rapportée et noyée dans la matière plastique.

4. Foret selon l'une quelconque des revendications 1 à 3, caractérisé en ce que la tige métallique (1) est munie d'un alésage longitudinal (1c) pour le passage d'un fluide lubrifiant, cet alésage (1c) communiquant avec au moins une gorge (2c) formée dans la partie active de coupe (2) pour amener le fluide jusqu'à la pointe du foret.

5. Foret selon la revendication 4, caractérisé en ce que la partie active de coupe (2) comporte des rainures hélicoïdales (2d) qui s'étendent selon l'axe longitudinal de ladite partie active de coupe (2), dans lesquelles débouchent la ou les gorges (2c).

6. Foret selon l'une quelconque des revendications 1 à 5, caractérisé en ce que la partie active de coupe (2) présente au niveau de son raccordement avec la tige métallique (1), une portée cylindrique de plus grand diamètre (2f).

## Claims

1. Drill for the insertion of a dental implant in particular, comprising a rotational drive portion which is in the form of a metal rod (1), intended to be-connected via a first end (1a) to an apparatus of the drilling machine type, and an active cutting portion (2) equipped with at least one sharp edge, characterized in that the active cutting portion (2) is made of plastic overmoulded on the second end of the rotational drive portion, and in that the said active cutting portion (2) has a metal insert (4) serving as a sharp edge.

2. Drill according to Claim 1, characterized in that the rotational drive portion (1) and the active cutting portion (2) comprise arrangements of complementary shapes (1b, 2a) capable of ensuring the integration of the two parts in terms of rotation.

3. Drill according to either one of Claims 1 and 2, characterized in that the metal insert of the active cutting portion (2) is a metal cutting tip (4) attached to and embedded in the plastic.

4. Drill according to any one of Claims 1 to 3, characterized in that the metal rod (1) is equipped with a longitudinal bore (1c) for the passage of a lubricating fluid, this bore (1c) communicating with at least one channel (2c) formed in the active cutting portion (2) for conveying the fluid as far as the point of the drill.

5. Drill according to Claim 4, characterized in that the active cutting portion (2) has helical flutes (2d) which extend along the longitudinal axis of the said active cutting portion (2) and into which the channel or channels (2c) open.

6. Drill according to any one of Claims 1 to 5, characterized in that the active cutting portion (2) has, in the area of its connection to the metal rod (1), a cylindrical span of greater diameter (2f).

## Patentansprüche

1. Bohrer, insbesondere zum Einsetzen eines dentalen Implantates, mit einem drehbar angetriebenem Teil, das die Gestalt einer metallischen Stange (1) aufweist, die dazu bestimmt ist, an einem ersten Ende (1a) mit einem Apparat nach Art einer Bohrmaschine verbunden zu werden, und mit einem Schneideteil (2), das wenigstens eine Schneidkante aufweist, dadurch **gekennzeichnet**, daß das Schneideteil (2) aus Kunststoff hergestellt ist, der um das zweite Ende des drehbar angetriebenen Teiles herum geformt ist, und daß das Schneideteil (2) einen metallischen Einsatz (4) umfaßt, der als Schneidkante dient.

2. Bohrer nach Anspruch 1, dadurch **gekennzeichnet**, daß das drehbar angetriebene Teil (1) und das Schneideteil (2) komplementäre Einrichtungen (1b, 2a) umfassen, die dafür geeignet sind, den Zusammenschluß der beiden drehbaren Teile zu sichern.

3. Bohrer nach einem der Ansprüche 1 oder 2, dadurch **gekennzeichnet**, daß der metallische Einsatz des Schneideteiles (2) ein Plättchen (4) aus Metall ist, das in den Kunststoff eingesetzt und eingelassen ist.

4. Bohrer nach einem der Ansprüche 1 bis 3, dadurch **gekennzeichnet**, daß die metallische Stange (1) mit einer longitudinalen Bohrung (1c) für den Durchgang eines Schmierfluides versehen ist, wobei die Bohrung (1c) mit wenigstens einer Auskehlung (2c) in Verbindung steht, die in dem Schneideteil (2) ausgebildet ist, um das Fluid bis zu der Spitze des Bohrers zu leiten.

5. Bohrer nach Anspruch 4, dadurch **gekennzeichnet**, daß das Schneideteil (2) schraubenförmige Nuten (2d) umfaßt, die sich längs der longitudinalen Achse des besagten Schneideteiles (2) erstrecken und in die die Auskehlung oder die Auskehlungen (2c) einmünden.

6. Bohrer nach einem der Ansprüche 1 bis 5, dadurch **gekennzeichnet**, daß das Schneideteil (2) auf Höhe seiner Verbindung mit der metallischen Stange (1) einen zylindrischen Bereich (2f) mit größerem Durchmesser aufweist.
